# EUROPEAN PATENT APPLICATION

(11) **EP 0 816 349 A1**
(43) Date of publication of application: **07.01.1998**
(21) Application number: 95941847.6
(22) Date of filing: 22.12.1995
(51) Int. Cl.: C07D 275/03, C07C 39/15, C07C 39/367, C07C 43/23, C09D 5/14, C09D 7/12

(54) **NOVEL CLATHRATE COMPOUND, PROCESS FOR PRODUCING THE SAME, AND COATING COMPOSITION CONTAINING SAID NOVEL CLATHRATE COMPOUND**

(30) Priority: 22.12.1994 JP 336543/94
(71) Applicant: ROHM AND HAAS COMPANY, Philadelphia, Pennsylvania 19106-2399 (US)
(72) Inventor: SUZUKI, Hiroshi, Ichihara-shi, Chiba 299-01 (JP); NAGAI, Hisao, Ichihara-shi, Chiba 299-01 (JP); KIKUCHI, Sachiko, Kawaguchi-shi, Saitama 333 (JP); YANAGI, Yoshikazu, Setagaya-ku, Tokyo 158 (JP)
(74) Representative: de Bruijn, Leendert C.
(86) International application number: JP9502636
(87) International publication number: WO9619465

(57) **Abstract**

A clathrate compound comprising a tetrakisphenol compound represented by general formula (I) as the host compound and a 2-alkyl-3(2H)-isothiazolone as the guest compound, wherein X represents (CH₂)ₙ; n represents 0, 1 or ; and R¹ to R⁸ represent each independently hydrogen, lower alkyl, halogeno, lower alkoxy or optionally substituted phenyl.

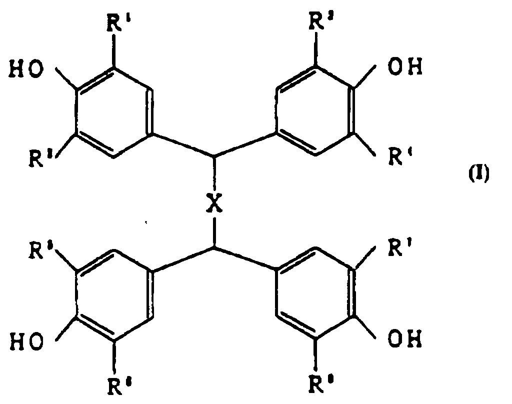

## Description

The present invention relates to a new clathrate compound, and particularly relates to a clathrate compound comprising tetrakisphenol as the host compound and 2-alkyl-3(2H) isothiazolone as the guest compound, its preparation method and a coating composition containing the new clathrate compound.

2-alkyl-3(2H) isothiazolones including 2-n-octyl-3(2H) isothiazolones are commonly used as industrial biocides for slime control, anti-foulant and other applications. They have a drawback of harsh effect to human bodies during application including high skin irritation and tendency to cause rash.

Further, many 2-alkyl-3(2H) isothiazolones are liquid and it makes difficult to use these compounds in the form of solid.

The object of the present invention is to provide a new clathrate compound having 2-alkyl-3(2H) isothiazolones as the guest compound and its preparation method and a coating composition containing the new clathrate compound as a means to obtain safe and solid 2-alkyl-3(2H) isothiazolones.

Having devoted themselves to their study to attain the above object, inventors successfully achieved the present invention with finding that, by directly adding a certain tetrakisphenol host compound to the liquid containing 2-alkyl-3(2H) isothiazolone for reaction or by mixing tetrakisphenol host compound powder and 2-alkyl-3(2H) isothiazolone powder for direct solid phase reaction or solid-liquid reaction, the new clathrate compound as defined in claim 1 can be quite effectively generated.

The tetrakisphenol used as the host compound in the present invention is the compound expressed by the formula [I] below.

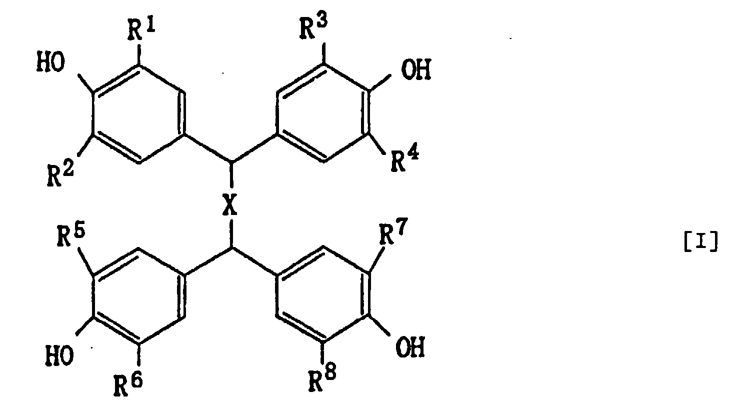

In the formula, X represents (CH₂)n where n may be 0, 1 or 2; and each of R¹ to R⁸ represents one component selected from the group consisting of hydrogen atom, lower-alkyl group, halogen atom, lower-alkoxy group and substitutable phenyl group.

The lower-alkyl group described is an alkyl group suitably having 1 to 5 carbon atoms. Preferred examples are a methyl, ethyl, propyl or isopropyl group. The lower-alkoxy group described above is an alkoxy group suitably having 1 to 5 carbon atoms and preferably is a methoxy, ethoxy, propoxy or isopropoxy group.

Specific examples of tetrakisphenols include 1,1,2,2-tetrakis (4-hydroxyphenyl) ethane, 1,1,2,2-tetrakis (3-methyl-4-hydroxyphenyl) ethane, 1,1,2,2-tetrakis (3-bromo-4-hydroxyphenyl) ethane, 1,1,2,2-tetrakis (3,5-dimethyl-4-hydroxyphenyl) ethane, 1,1,2,2-tetrakis (3-t-butyl-4-hydroxyphenyl) ethane, 1,1,2,2-tetrakis (3-chloro-4-hydroxyphenyl) ethane, 1,1,2,2-tetrakis (3,5-dichloro-4-hydroxyphenyl) ethane, 1,1,2,2-tetrakis (3-fluoro-hydroxyphenyl) ethane, 1,1,2,2-tetrakis (3,5-difluoro-4-hydroxyphenyl) ethane, 1,1,2,2-tetrakis (3-methoxy-4-hydroxyphenyl) ethane, 1,1,2,2-tetrakis (3,5-dimethoxy-4-hydroxyphenyl) ethane, 1,1,2,2-tetrakis (3-chloro-5-methyl-4-hydroxyphenyl) ethane, 1,1,2,2-tetrakis (3-chloro-5-methyl-4-hydroxyphenyl) ethane, 1,1,2,2-tetrakis (3-chloro-5-phenyl-4-hydroxyphenyl) ethane, 1,1,2,2-tetrakis [(hydroxy-3-phenyl) phenyl] ethane, 1,1-bis (4-hydroxyphenyl)-2,2-bis(3-methyl-4-hydroxyphenyl) ethane, 1,1,3,3-tetrakis(4-hydroxyphenyl) propane, 1,1,3,3-tetrakis (3-methyl-4-hydroxyphenyl) propane, 1,1,3,3-tetrakis (3,5-dimethyl-4-hydroxyphenyl) propane, 1,1,3,3-tetrakis (3-chloro-4-hydroxyphenyl) propane, 1,1,3,3-tetrakis (3,5-dichloro-4-hydroxyphenyl) propane, 1,1,3,3-tetrakis (3-fluoro-4-hydroxyphenyl) propane, 1,1,3,3-tetrakis (3,5-difluoro-4-hydroxyphenyl) propane, 1,1,3,3-tetrakis (3-phenyl-4-hydroxyphenyl) propane, 1,1,4,4-tetrakis (4-hydroxyphenyl) butane, 1,1,4,4-tetrakis (3-methyl-4-hydroxyphenyl) butane, 1,1,4,4-tetrakis (3,5-dimethyl-4-hydroxyphenyl) butane, 1,1,4,4-tetrakis (3-chloro-4-hydroxyphenyl) butane, 1,1,4,4-tetrakis (3,5-dichloro-4-hydroxyphenyl) butane, 1,1,4,4-tetrakis (3-fluoro-4-hydroxyphenyl) butane and 1,1,4,4-tetrakis (3,5-difluoro-4-hydroxyphenyl) butane.

The alkyl group in 2-alkyl-3(2H) isothiazolone, which serves as the guest compound in the present invention, is preferably a straight-chain or branched-chain alkyl group having 1 to 20 carbons.

Specific examples of 2-alkyl-3(2H) isothiazolones which can be used in this invention include 2-methyl-3(2H) isothiazolone, 2-ethyl-3(2H) isothiazolone, 2-isopropyl-3(2H) isothiazolone, 2-n-propyl-3(2H) isothiazolone, 2-n-butyl-3(2H) isothiazolone, 2-sec-butyl-3(2H) isothiazolone, 2-t-butyl-3(2H) isothiazolone, 2-n-pentyl-3(2H) isothiazolone, 2-neopentyl-3(2H) isothiazolone, 2-n-hexyl-3(2H) isothiazolone, 2-c-hexyl-3(2H) isothiazolone, 2-n-heptyl-3(2H) isothiazolone, 2-n-octyl-3(2H) isothiazolone, 2-n-nonyl-3(2H) isothiazolone, 2-n-decyl-3(2H) isothiazolone, 2-n-undecyl-3(2H) isothiazolone and 2-n-dodecyl-3(2H) isothiazolone.

The new clathrate compound of the present invention can be obtained by mixing a dispersion or solution of 2-alkyl-3(2H) isothiazolone and tetrakisphenol as the host compound and stirring them at a temperature from the room temperature to 100°C for a couple of minutes to a couple of hours for reaction so that 2-alkyl-3(2H) isothiazolone is easily clathrated to the host compound.

Alternatively, the new clathrate compound of the present invention can be obtained by mixing 2-alkyl-3(2H) isothiazolone and tetrakisphenol powder as the host compound at a temperature from the room temperature to 60°C for a couple of minutes to a couple of hours for solid phase reaction or solid-liquid reaction so that 2-alkyl-3(2H) isothiazolone is easily clathrated into the host compound.

The characteristics of the new clathrate compound and its preparation method of this invention are summarized below:
1. The clathrate compound obtained according to the present invention is almost free from skin irritating property against human bodies. It is thus extremely easily handled.
2. Heating treatment to the clathrate compound becomes possible since the releasing temperature of 2-alkyl-3(2H) isothiazolone from the clathrate compound (for example, in case 2-n-octyl-3(2H) isothiazolone is used as the guest compound, though the guest compound is in the form of liquid, releasing temperature from the clathrate compound is above 190°C).
3. Since the clathrate compound of the present invention is in the form of solid, it can be used as a component of ship-bottom anti-foulant in the form of solvent-dispersion.
4. By clathrating, 2-alkyl-3(2) isothiazolones including liquid material of 2-alkyl-3(2H) isothiazolone can be solidified and can be used as solid biocide and solid slime-control agent.
5. A new clathrate compound comprising tetrakisphenols as the host compound and 2-alkyl-3(2H) isothiazolone as the guest compound can be easily prepared.

The new clathrate compound of this invention is particularly suitably used for coating compositions.

This compound can be used for water-based paints and solvent-based paints. By adding an appropriate amount of this clathrate compound to a water-soluble polymer compound such as casein-formalin copolymers, water-soluble vinyl-based and acryl-based polymers, water-soluble vegetable oils and polyester resins, or to a latex such as methyl acrylate-styrene, vinyl acetate-styrene, vinyl acetate-methyl methacrylate, vinyl acetate-maleic anhydride, styrene-butadiene, vinyl acetate-methyl acrylate and vinyl choride-vinyl chloride-vinyl acetate a coating composition can be prepared.

A preferable amount to be added can be determined suitably according to the application and desired performance of a coating composition to be prepared and can also be determined easily by those skilled in the art according to well-known methods. The new clathrate compound of this invention to be used for coating compositions does not irritate skin and eyes, does not have an irritating odor, and can be handled easily. Compared with the case of solely using 2-alkyl-3(2H) isothiazolone, the 2-alkyl-3(2H) isothiazolone can be used in a larger amount as an active component. Therefore, a coating composition with improved safety and high effect can be obtained.

The coating composition obtained can be applied to various substrates such as fiber, paper, leather, wood, ceramics and metal, and can be used extensively, such as as an interior or exterior paint. The clathrate compound prepared by this invention is free from the problems such as irritation to skin and eyes and the irritating odor involved in handling the 2-alkyl-3(2H) isothiazolone and can be used easily. And, clathration allows to solidify 2-n-alkyl-3(2H) isothiazolone, such as liquid 2-n-octyl-3(2H) isothiazolone, and to use it as a solid germicide and a solid slime controlling agent.

In addition to the above coating compositions, the clathrate compound of this invention can be used specifically as follows: a slime preventing agent in cooling water systems, an algaecide for swimming pools, a disinfectant, a slime preventing agent for paper making processes, a fungicide for texture, a fungicide for leather, a bactericide for soap, an antiseptic agent for metalworking fluid, an antiseptic agent for paint, and a sanitary agent for texture.

### Brief description of the drawings:

Figure 1 shows an IR spectrum of the clathrate compound obtained in Example 1.

Figure 2 is an IR spectrum of a guest compound 2-n-octyl-3(2H) isothiazolone.

This invention will be described in detail by means of the following examples. However, this invention is not limited by these examples.

### Example 1

Into 10 ml of water, 1.1 g of 2-n-octyl-3(2H) isothiazolone (a product of Rohm & Haas) was dispersed, and 1 g of 1,1,2,2-tetrakis (4-hydroxyphenyl) ethane was added to prepare a suspension, which was then stirred at 60 degrees C for one hour. A resulting reaction solution was left to stand at room temperature for 24 hours, and subjected to filtration under reduced pressure to obtain a solid substance. The solid substance was vacuum-dried at room temperature to obtain a light-yellow powdered clathrate compound consisting of 2-n-octyl-3(2H) isothiazolone and 1,1,2,2-tetrakis (4-hydroxyphenyl) ethane at a ratio of 2 to 1 (mole ratio).

The obtained clathrate compound was checked by the TG-DTA analysis and the IR analysis.

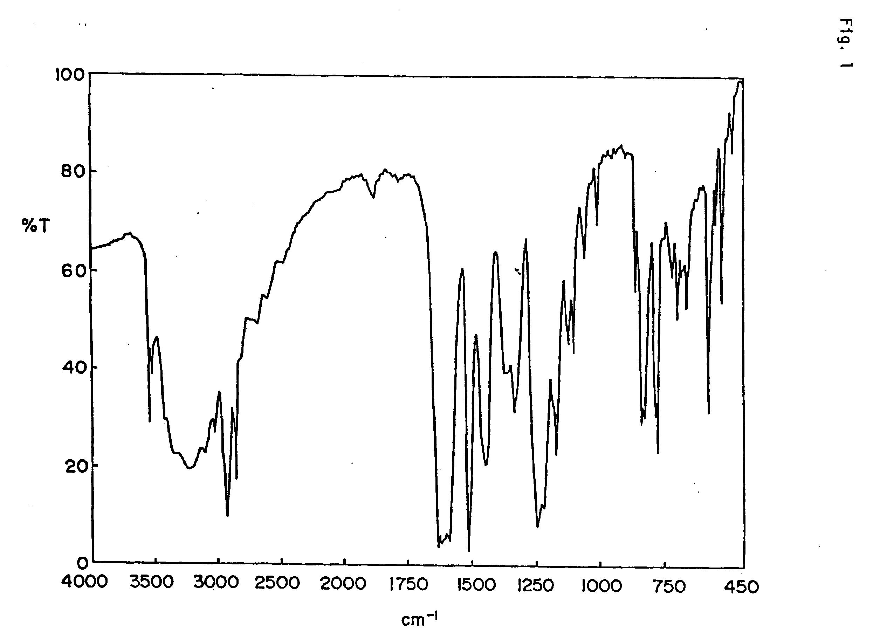

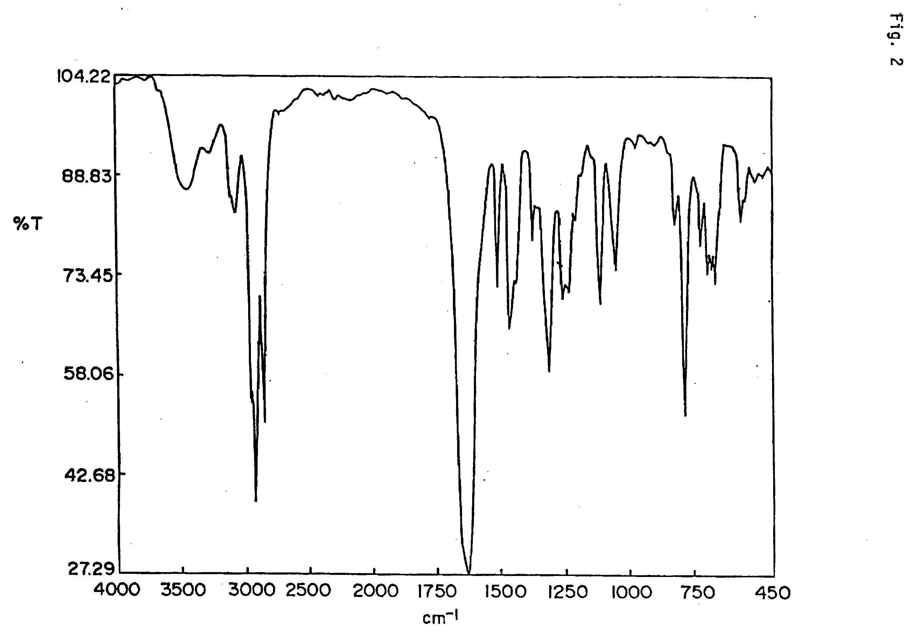

Figure 1 shows an IR spectrum of the clathrate compound (KBr method), and Figure 2 shows an IR spectrum of a guest compound, 2-n-octyl-3(2H) isothiazolone (KBr method).

### Example 2

To 1.1 g of 2-n-octyl-3(2H) isothiazolone which was heated to 60 degrees C, 1.0 g of 1,1,2,2-tetrakis (4-hydroxyphenyl) ethane at a ratio of 2 to 1 (mole ratio).

The obtained clathrate compound was checked by the TG-DTA analysis and the IR analysis. It was confirmed that the same substance was prepared as in Example 1.

Table 1 shows the production conditions to prepare the clathrate compounds in Examples 1 and 2, the mole ratios of the guests and the hosts of the obtained clathrate compounds, and the temperature to release the guest organic compound.

**Table 1**

| Example | Reaction conditions | | | Guest/host mole ratio | Guest releasing temperature (°C) | Melting point of clathrate (°C) |
|---|---|---|---|---|---|---|
| | Solvent | Temperature (°C) | Time (min) | | | |
| 1 | Water | 60 | 60 | 2.0 | 194 | 61 |
| 2 | Not used | 60 | 10 | 2.0 | 196 | 63 |

### Example 3

To an acrylic emulsion, water-based matte paint (Toriopainter), produced by Nihon Tokushu Toryo Co., Ltd., prescribed amounts of a 45% propylene glycol solution of 2-n-octyl-3(2H) isothiazolone (test substance A) and the clathrate compound consisting of 2-n-octyl-3(2H) isothiazolone and 1,1,2,2-tetrakis (4-hydroxyphenyl) ethane at a ratio of 2 to 1 (mole ratio) obtained in Example 1 (test substance B) were added. The resulting substance was applied in an appropriate amount two times to release liner paper. The paper was air-dried for one week to obtain an ordinary paint film. The film separated from the paper was weighed and placed in a 70-ml. mayonnaise bottle and 50 ml of water was added thereto. Then the solution was subjected to a shaking culture at 25 degrees C. Samples were collected at certain time intervals and determined by HPLC.

Table 2 shows the details of the test film samples, and Table 3 shows the eluted 2-n-octyl-3(2H) isothiazolone in mg and percentage.

It is seen from Table 3 that the 2-n-octyl-3(2H) isothiazolone eluted from the clathrate compound of this invention is rather small in ratio.

**Table 2**

| Test substance | Active component in wet paint (ppm) | Solid content in paint (%) | Dried film (g) | Active component in film (ppm) | Total active component in film sample (mg) |
|---|---|---|---|---|---|
| A | 1350 | 32 | 1.9184 | 4218 | 8.09 |
| B | 3000 | 32 | 2.144 | 9375 | 20.10 |

**Table 3**

| Test substance | Just after | 4 hrs | 8 hrs | 24 hrs | 48 hrs | 72 hrs | 96 hrs | 168 hrs |
|---|---|---|---|---|---|---|---|---|
| A | | | | | | | | |
| Eluted amount mg | 1.80 | 2.25 | 2.40 | 2.57 | 2.85 | 2.88 | 2.92 | 2.94 |
| Eluation % | 22.19 | 27.75 | 29.60 | 31.77 | 35.23 | 35.54 | 36.03 | 36.34 |

| B | | | | | | | | |
|---|---|---|---|---|---|---|---|---|
| Eluted amount mg | 3.54 | 4.55 | 4.58 | 4.985 | 5.75 | 5.815 | 6.035 | 6.405 |
| Eluation % | 17.61 | 22.64 | 22.79 | 24.80 | 28.61 | 28.93 | 30.02 | 31.87 |

### Example 4

An acrylic binder for water-based gloss paint and another acrylic binder for water-based matte paint which contain, as a pigment, titanium dioxide, CR-50, produced by Ishihara Sangyo Kaisha, Ltd. were used. To prepare paints, test substances A and B were added in 4000 ppm a.l., and their adding amounts were adjusted by sequentially diluting with a non-addition paint. The paints were applied, in 25 g per one square meter, to veneers (50 mm x 100 mm) whose surface was magnolia two times at intervals of four hours, and air-dried for one week or more to prepare film samples. The film samples were repeatedly used three times. Test fungi were inoculated on the samples and cultivated at 30°C for 37 days.

The inoculated fungi are as follows.

| I.D. | Name of fungus |
|---|---|
| IFO#6345 | Penicillium funiculosum |
| IFO#6353 | Aureobasidium pullulans |
| IFO#6342 | Aspergillus niger |

Mildewproof evaluation indexes were set as follows.

| Fungus growth rate | Contamination level | Fungus growth evaluation |
|---|---|---|
| 0 | Nil | 0 |
| T | Trace | < 5% |
| 1+ | Very little | > 10% |
| 2+ | Little | 25% +/- |
| 3+ | Moderate | 50% +/- |
| 4+ | Many | > 50% |

**Table 4**

| Test substance | Dosage (ppm, active component) | Fungus growth rate | |
|---|---|---|---|
| | | Gloss paint | Matte paint |
| B | 1000 | 1+ | 1+ |
| B | 2000 | 1+ | 2+** |
| B | 3000 | T | 1+ |
| B | 4000 | T | T |
| A | 1000 | 4+ | 3+ |
| Blank | 1 | 4+ | 4+ |

| | | | |
|---|---|---|---|
| **: Much more growth was observed sometimes. | | | |

Films of the paints prepared by adding 1000 ppm a.l. of the clathrate compound prepared in Example 1 to water-based gloss and matte paints were confirmed having a very superior mildewproof effect to those of the paint containing 2-n-octyl-3(2H) isothiazolone in equal weight.

### Test Example

A 45% propylene glycol solution of 2-n-octyl-3(2H) isothiazolone (test substance A) and the clathrate compound consisting of 2-n-octyl-3(2H) isothiazolone and 1,1,2,2-tetrakis (4-hydroxyphenyl) ethane at a ratio of 2 to 1 (mole ratio) obtained in Example 1 (test substance B) were tested for safety by the following method.

### 1. Preparation of test substances

Each substance was not treated. A solution of 2-n-octyl-3(2H) isothiazolone in a state of yellow liquid and the clathrate compound in a state of crystalline powder of Example 1 were used as they were.

### 2. Used animal

Rabbit: New Zealand White, male

### 3. Dosage

The rabbits had their back hair cut a day before testing. To a round lint cloth having a diameter of 1.5 cm, 0.1 g or 0.1 ml of each substance was applied using a little amount of ion-exchanged water, if necessary, and the cloth was put on the skin in good condition. A 6 x 6 cm gauze was put on the cloth and a breathing bandage was used to fix them. Thus, a semi-closed patch test was conducted for four hours. After removing the cloth, the remained test substance was wiped off with water. Two rabbits were used for each test substance.

### 4. Observation and evaluation

Skin damages were observed and evaluated one hour and seven days after removing the cloth, then scored as shown in Table 5. An average of damages for three days after removing the cloth was calculated and indicated as a skin primary irritation level index (hereafter called "PII").

### 5. Results

Table 6 shows the results.

When 2-n-octyl-3(2H) isothiazolone was used, the highest point 4 was recorded for erythema and dropsy. Later the dropsy was reduced but necroses were observed. These necroses did not heal even after seven days. It had the PII of 6.8 and was judged to be a very irritating (caustic) substance.

On the other hand, the clathrate was scored 0 to 2, and erythema and dropsy healed in six days. It had the PII of 1.9 and was judged to be a slightly irritating substance.

It is seen from the test results that clathration with TEP and pulverizing sharply reduced irritation to skin and improved safety.

**Table 5**

| Erythema and scab | |
|---|---|
| No erythema | 0 |
| Very slight erythema (barely recognizable) | 1 |
| Clear erythema | 2 |
| Moderate to severe erythema | 3 |
| Severe erythema (beet redness) to formation of slight scab (damage to a depth) | 4 |
| Maximum point No dropsy | 4 0 |

| Dropsy | |
|---|---|
| No dropsy | 0 |
| Very slight dropsy (barely recognizable) | 1 |
| Slight dropsy (clear edge due to an obvious protuberance can be observed) | 2 |
| Moderate dropsy (about 1-mm high protuberance) | 3 |
| Severe dropsy (about 1-mm high protuberance and an expanse exceeding the exposed area) | 4 |
| Maximum point | 4 |

**Table 6**

| Test substance and animal No. | | Day-varying score | | | | | | | | | | | | Average score to the third day and note | |
|---|---|---|---|---|---|---|---|---|---|---|---|---|---|---|---|
| | | Erythema/scab | | | | | | Dropsy | | | | | | | |
| | | (hour) | | | (days) | | | (hour) | | | (days) | | | | |
| | | 1 | 1 | 2 | 3 | 6 | 7 | 1 | 1 | 2 | 3 | 6 | 7 | | |
| A | 01 | 4 | 4 | 4 | 4 | 4 | 4 | 4 | 4 | 2 | 2 | 1 | 1 | 28 / 4 = 7.0 | 6.8* Necroses observed |
| | 02 | 4 | 4 | 4 | 4 | 4 | 4 | 4 | 4 | 1 | 1 | 1 | 1 | 26 / 4 = 6.5 | |
| B | 01 | 1 | 2 | 2 | 2 | 0 | - | 1 | 1 | 0 | 0 | 0 | - | 9 / 4 = 2.2 | 1.9* |
| | 02 | 1 | 2 | 2 | 1 | 0 | - | 0 | 0 | 0 | 0 | 0 | - | 6 / 4 = 1.5 | |

| | | | | | | | | | | | | | | | |
|---|---|---|---|---|---|---|---|---|---|---|---|---|---|---|---|
| *: Light ≤ 2, Medium = 2 to 5, Severe ≥ 5 -: Not observed | | | | | | | | | | | | | | | |

## Claims

1. A clathrate compound comprising as the host compound a tetrakisphenol expressed by formula [I] 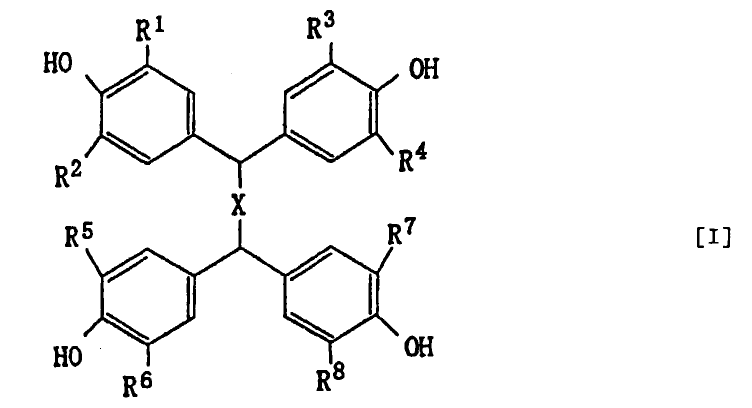 wherein X represents (CH₂)n, where n may be 0, 1 or 2; and each of R¹ to R⁸ represents one component selected from the group consisting of hydrogen atom, lower-alkyl group, halogen atom, lower-alkoxy group and substitutable phenyl group,
and 2-alkyl-3(2H) isothiazolone as the guest compound.

2. A process for the preparation of the clathrate compound according to claim 1, wherein this compound is obtained through reaction between tetrakisphenol expressed by formula [I] and 2-alkyl-3(2H) isothiazolone.

3. A coating composition containing the clathrate compound according to claim 1.
